(19) Europäisches Patentamt / European Patent Office / Office européen des brevets

(11) **EP 4 394 025 A1**

(12) **EUROPEAN PATENT APPLICATION**
published in accordance with Art. 153(4) EPC

(43) Date of publication:
**03.07.2024 Bulletin 2024/27**

(21) Application number: **22861433.5**

(22) Date of filing: **25.08.2022**

(51) International Patent Classification (IPC):
*C12M 1/00* (2006.01)   *B32B 7/12* (2006.01)
*B32B 27/00* (2006.01)   *B32B 27/36* (2006.01)
*C09J 7/35* (2018.01)   *C09J 167/00* (2006.01)
*C12N 1/00* (2006.01)   *C12N 5/00* (2006.01)

(52) Cooperative Patent Classification (CPC):
**B32B 7/12; B32B 27/00; B32B 27/36; C09J 7/35;
C09J 167/00; C12M 1/00; C12N 1/00; C12N 5/00**

(86) International application number:
**PCT/JP2022/032043**

(87) International publication number:
**WO 2023/027145 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.08.2021 JP 2021138137**

(71) Applicant: **TOKYO OHKA KOGYO CO., LTD.
Kawasaki-shi, Kanagawa 211 0012 (JP)**

(72) Inventors:
• **KAMIZONO, Takashi
  Kawasaki-shi, Kanagawa 211-0012 (JP)**
• **FUJIMOTO, Takashi
  Kawasaki-shi, Kanagawa 211-0012 (JP)**
• **AKABANE, Takafumi
  Tokyo 104-0032 (JP)**
• **SATOH, Taku
  Tokyo 104-0032 (JP)**
• **MORIGUCHI, Hiroyuki
  Tokyo 104-0032 (JP)**

(74) Representative: **Plasseraud IP
66, rue de la Chaussée d'Antin
75440 Paris Cedex 09 (FR)**

(54) **CELL CULTURE CHIP, CELL CULTURE DEVICE, METHOD FOR PRODUCING CELL CULTURE CHIP, AND METHOD FOR CULTURING CELLS**

(57)   A chip for cell culture, including a laminate having a flow path structure inside, in which respective members constituting respective layers of the laminate are adhered by adhesive layers, and the adhesive layer includes a first adhesive layer formed of an adhesive that contains a polyester-based resin having a glass transition temperature of 37°C or higher and 120°C or lower.

**EP 4 394 025 A1**

**Description**

[Technical Field]

The present invention relates to a chip for cell culture, a device for cell culture, a method for producing a chip for cell culture, and a method for culturing cells.
Priority is claimed on Japanese Patent Application No. 2021-138137, filed August 26, 2021, the content of which is incorporated herein by reference.

[Background Art]

In monolayer culture which has been used for cell assays in the related art, an environment surrounding the cells is greatly different from that in vivo. Therefore, there often occurs a problem in that in cultured cells in the monolayer culture, most of functions expressed by cells in vivo are lost. It is expected that the problem is solved by advances in microfabrication technology and three-dimensional culture technology in recent years so that cell assays are improved in terms of throughput and reliability at the same time. In particular, the concept of organ-on-a-chip, which handles a chip for cell culture provided with a microfluidic device that reproduces a physiological three-dimensional culture environment in vitro as one organ, has spread, and a research in consideration of the applications for drug development has been widely developed worldwide. Furthermore, the concept of a body-on-a-chip, which aims to reproduce individual responses by connecting a plurality of organ models reconstructed in vitro with microflow paths and the like, has been proposed and has been attracting attention rapidly. Such a system is also referred to as a microphysiolocical system (MPS).
As the chip for cell culture as described above, there has been proposed a chip for cell culture, provided with a microflow path structure body having a hollow structure, which is obtained by thermocompression-bonding a substrate in which a flow path for cell culture is formed to a top plate blocking the flow path for cell culture through an adhesive layer which is a polyester-based resin having a Tg of 5°C or higher (see, for example, Patent Document 1).

[Citation List]

[Patent Document]

[Patent Document 1]
Japanese Unexamined Patent Application, First Publication No. 2018-102236

[Summary of Invention]

[Technical Problem]

The human mimicry systems as described above have attracted attention as a method for evaluating the efficacy and the safety of a drug in vitro. In evaluation of the drug, cells are cultured in the presence of a drug, and an effect of the drug on the cell is observed in a chip for cell culture constituting an MPS.
As the chip for cell culture used for the evaluation of the drug, there is a demand for conditions that there is no leakage of a cell medium under a perfusion condition of around 37°C close to the human body temperature; the culture and the observation of cells to be evaluated are possible; and there is a less reduction in the drug. In order to satisfy the condition of a less reduction in the drug, it is necessary to suppress the sorption of the drug onto an adhesive layer in contact with a flow path during the perfusion of the drug solution. However, in a chip for drug culture in the related art, it cannot be said to be sufficient for suppressing the sorption of a drug onto an adhesive layer.
The present invention has been made in view of the circumstances, and an object of the present invention is to provide a chip for cell culture, which can suppress the sorption of a drug onto an adhesive layer; a device for cell culture, including the chip for cell culture; a method for producing the chip for cell culture; and a method for culturing cells, using the chip for cell culture.

[Solution to Problem]

In order to accomplish the object, the present invention adopted the following configurations.
A first aspect of the present invention is a chip for cell culture, including a laminate having a flow path structure inside, in which respective members constituting respective layers of the laminate are adhered by adhesive layers, and the adhesive layer includes a first adhesive layer formed of an adhesive that contains a polyester-based resin having a glass transition temperature of 37°C or higher and 120°C or lower.

A second aspect of the present invention is a device for cell culture, including the chip for cell culture of the first aspect.

A third aspect of the present invention is a method for producing a chip for cell culture, including a laminate having a flow path structure inside, the method including a step A of laminating respective members constituting respective layers of the laminate through adhesive layers; and a step B of adhering the respective laminated members by the adhesive layers, in which the adhesive layer includes a first adhesive layer formed of an adhesive that contains a polyester-based resin having a glass transition temperature of 37°C or higher and 120°C or lower.

A fourth aspect of the present invention is a method for culturing cells, including a step of culturing cells in the presence of a drug within the flow path of the chip for cell culture of the first aspect.

[Advantageous Effects of Invention]

According to the present invention, there are provided a chip for cell culture, which can suppress the sorption of a drug onto an adhesive layer; a device for cell culture, including the chip for cells; a method for producing the chip for cell culture; and a method for culturing cells, using the chip for cell culture.

[Brief Description of Drawings]

FIG. 1 is a perspective view of a chip for cell culture according to an embodiment.
FIG. 2 is a dissembling view of the chip for cell culture of FIG. 1.
FIG. 3 is a cross-sectional view of the chip for cell culture of FIG. 1, taken along a cut line III-III.
FIG. 4 is a cross-sectional view of the chip for cell culture of FIG. 1, taken along a cut line IV-IV.
FIG. 5 is a graph showing an example of the thickness occupied by each layer in each substrate in the chip for cell culture according to an embodiment.
FIG. 6 is a graph showing an example of the thickness occupied by each layer in each substrate in the chip for cell culture according to an embodiment.
FIG. 7 is a schematic view showing an example of cell culture using the chip for cell culture according to an embodiment.

[Description of Embodiments]

Hereinafter, embodiments of the present invention will be described in detail with reference to the drawings depending on cases. In the drawings, the same or corresponding portions will be denoted by the same or corresponding reference numerals, and overlapping descriptions will be omitted. Dimensional ratios in each drawing are exaggerated for explanation and do not necessarily match the actual dimensional ratios.

(Chip for Cell Culture)

A first aspect of the present invention is a chip for cell culture, including a laminate having a flow path structure inside. The respective members constituting the respective layers of the laminate are adhered by adhesive layers. The adhesive layer includes a first adhesive layer formed of an adhesive that contains a polyester-based resin having a glass transition temperature of 37°C or higher and 120°C or lower.

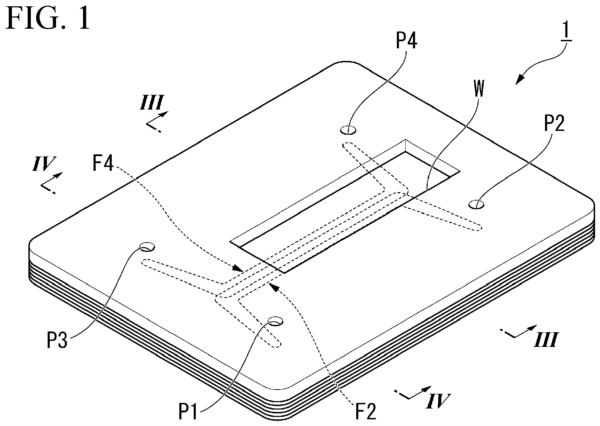

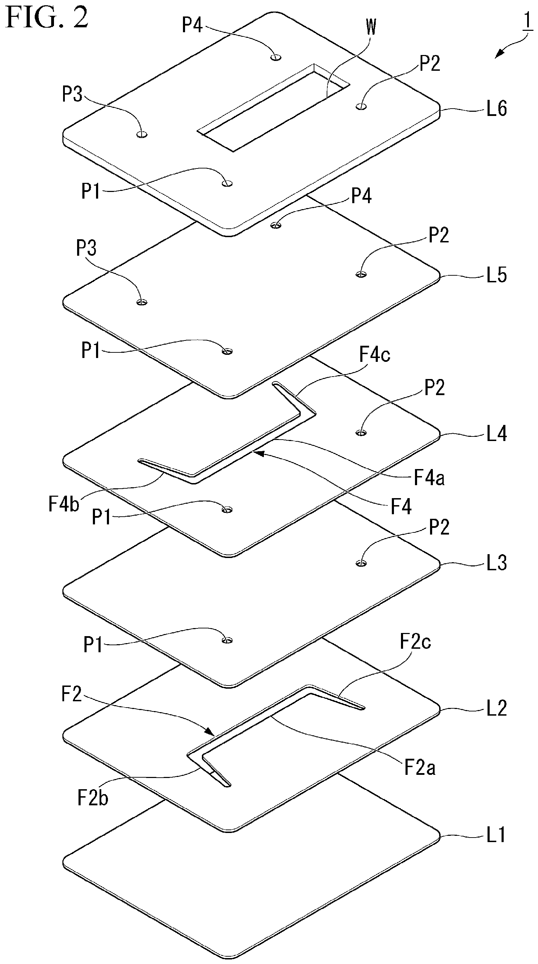

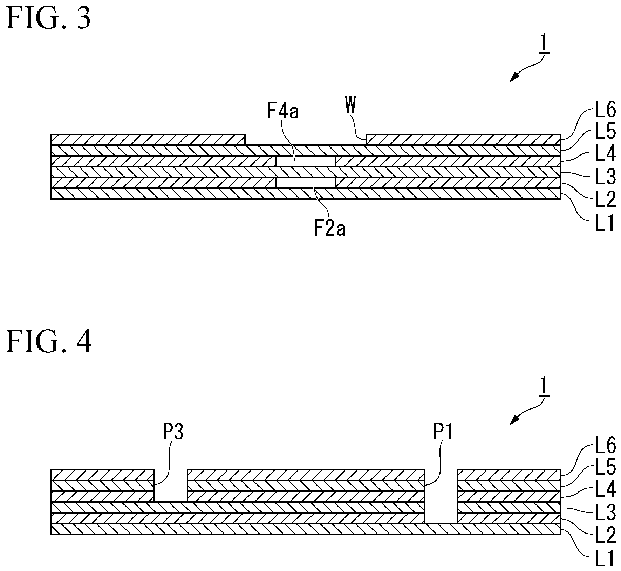

FIG. 1 is a perspective view of a chip for cell culture according to an embodiment. FIG. 2 is a dissembling view of a chip 1 for cell culture of FIG. 1. FIG. 3 is a cross-sectional view of the chip 1 for cell culture of FIG. 1, taken along a cut line III-III. FIG. 4 is a cross-sectional view of the chip 1 for cell culture of FIG. 1, taken along a cut line IV-IV.

The chip 1 for cell culture is a laminate having a flow path structure inside. The laminate of the chip 1 for cell culture is composed of a bottom plate substrate L1, a first flow path substrate L2, a porous membrane L3, a second flow path substrate L4, a top plate substrate L5, and a cover substrate L6. The bottom plate substrate L1, the first flow path substrate L2, the porous membrane L3, the second flow path substrate L4, the top plate substrate L5, and the cover substrate L6 are laminated in this order.

A first flow path F2 is formed in the first flow path substrate L2. A second flow path F4 is formed in the second flow path substrate L4. By laminating the respective substrates as described above, a flow path structure consisting of the first flow path F2 and the second flow path F4 is formed inside the chip 1 for cell culture.

The chip 1 for cell culture is provided with a port P1 as a drug solution introduction port into the first flow path F2, a port P2 as a drug solution discharge port from the first flow path F2, a port P3 as a drug solution introduction port into the second flow path F4, and a port P4 as a drug solution discharge port from the second flow path F4.

The first flow path F2 is formed of a first central flow path F2a, a first introduction flow path F2b that connects the port P1 and the first central flow path F2a, and a first discharge flow path F2c that connects the port P2 and the first central flow path F2a. A drug solution introduced from the port P1 is discharged from the port P2 through the first introduction flow path F2b, the first central flow path F2a, and the first discharge flow path F2c.

The second flow path F4 is formed of a second central flow path F4a, a second introduction flow path F4b that connects the port P3 and the second central flow path F4a, and a second discharge flow path F4c that connects the port P4 and the second central flow path F4a. A drug solution introduced from the port P3 is discharged from the port P4 through the second introduction flow path F4b, the second central flow path F4a, and the second discharge flow path F4c.

The first central flow path F2a and the second central flow path F4a are arranged so that at least parts of the flow paths overlap each other. The first central flow path F2a and the second central flow path F4a are separated by a porous membrane M. In the chip 1 for cell culture, the first central flow path F2a is a lower layer flow path located in the lower part of the porous membrane M. The second central flow path F4a is an upper flow path located in the upper part of the porous membrane M.

The flow path widths of the first central flow path F2a and the second central flow path F4a can be appropriately set according to the purpose of the chip 1 for cell culture. Examples of the flow path width include 1 $\mu$m to 10,000 $\mu$m, 10 $\mu$m to 8,000 $\mu$m, 100 $\mu$m to 5,000 $\mu$m, and 500 $\mu$m to 3,000 $\mu$m.

The cover substrate L6 is provided with an opening part W so that the first central flow path F2a and the second central flow path F4a can be observed.

<Adhesive Layer>

The respective members constituting the chip for cell culture are adhered by adhesive layers. The adhesive layer includes a first adhesive layer formed of an adhesive that contains a polyester-based resin having a glass transition temperature of 37°C or higher and 120°C or lower.

<<First Adhesive Layer>>

The first adhesive layer is formed of an adhesive (hereinafter also referred to as a first adhesive) that contains a polyester-based resin having a glass transition temperature (hereinafter also referred to as a "Tg") of 37°C or higher and 120°C or lower (hereinafter also referred to as a "resin (A)").

Polyester-Based Resin at 37°C or Higher and 120°C or Lower (Resin (A))

The resin (A) is not particularly limited as long as it is a polyester-based resin having a Tg of 37°C or higher and 120°C or lower. A lower limit value of the Tg of the resin (A) is preferably 40°C or higher, more preferably 45°C or higher, still more preferably 50°C or higher, and particularly preferably 55°C or higher. An upper limit value of the Tg of the resin (A) is preferably 110°C or lower, more preferably 100°C or lower, still more preferably 95°C or lower, and particularly preferably 90°C or lower. The Tg of the resin (A) is preferably 40°C to 110°C, more preferably 45°C to 100°C, still more preferably 50°C to 95°C, and particularly preferably 55°C to 90°C.

The "glass transition temperature (Tg)" is a temperature at which glass transition occurs. The Tg can be determined as a temperature at which the tangents intersect with each other at a bending inflection point of the measured temperature obtained by differential scanning calorimetry (DSC) under the condition of a temperature rising rate of 20°C/min.

The "polyester-based resin" refers to a resin including an ester bond (-CO-O-) in the main chain. Examples of the polyester-based resin include a polyester resin and a polyester urethane resin. Examples of the resin (A) include a polyester resin having a Tg of 37°C or higher and 120°C or lower, and a polyester urethane resin having a Tg of 37°C or higher and 120°C or lower.

The polyester resin can be obtained by copolymerizing a polyvalent carboxylic acid and a polyol. Examples of a commercially available product of the polyester resin having a Tg of 37°C or higher and 120°C or lower include "VYLON (registered trademark)" series (manufactured by Toyobo Co., Ltd.), "ELITEL (registered trademark)" series (manufactured by Unitika Ltd.), and G7 KuranBeter manufactured by Kurabo Industries Ltd.

Examples of the polyester resin having a Tg of 37°C or higher and 120°C or lower among the "VYLON (registered trademark)" series (manufactured by Toyobo Co., Ltd.) include VYLON 103 (47°C), VYLON 200 (67°C), VYLON 220 (53°C), VYLON 226 (65°C), VYLON 240 (60°C), VYLON 245 (60°C), VYLON 270 (67°C), VYLON 280 (68°C), VYLON 290 (72°C), VYLON 296 (71°C), VYLON 300 (7°C), VYLON 600 (47°C), VYLON GK110 (50°C), VYLON GK250 (60°C), VYLON GK360 (56°C) VYLON GK640 (79°C), VYLON GK810 (46°C), and VYLON GK880 (84°C) The numbers in parentheses indicate Tg.

Examples of the polyester resin having a Tg of 37°C or higher and 120°C or lower among "ELITEL (registered trademark)" series (manufactured by Unitika Ltd.) include ELITEL UE-3210 (45°C), ELITEL UE-9200 (65°C), ELITEL UE-3600 (75°C), ELITEL UE-9800 (85°C), ELITEL UE-9900 (101°C), ELITEL UE-3320 (40°C), ELITEL UE-9820 (39°C), ELITEL UE-3350 (52°C), and ELITEL UE-3380 (60°C). The numbers in parentheses indicate Tg.

Examples of the number-average molecular weight (Mn) of the polyester resin as the resin (A) include 5,000 to 100,000. The number-average molecular weight (Mn) is determined based on polystyrene conversion according to gel permeation chromatography (GPC).

The polyester urethane resin is a resin including an ester bond (-CO-O-) and a urethane bond (-NCO-) in the main chain. The polyester urethane resin can be obtained by copolymerizing a polyvalent carboxylic acid, a polyol, and a polyisocyanate. Alternatively, it can be obtained by crosslinking a polyester resin with a polyisocyanate.

Examples of a commercially available product of the polyester urethane resin having a Tg of 37°C or higher and 120°C or lower include "VYLON (registered trademark) UR" series (manufactured by Toyobo Co., Ltd.).

In the "VYLON (registered trademark) UR" series (manufactured by Toyobo Co., Ltd.), examples of the polyester urethane resin having a Tg of 37°C or higher and 120°C or lower include VYLON UR-1350 (46°C), VYLON UR-1400 (83°C), VYLON UR-4800 (106°C), and VYLON UR-3600 (40°C). The numbers in parentheses indicate Tg.

Examples of the number-average molecular weight (Mn) of the polyester urethane resin as the resin (A) include 5,000 to 100,000.

The polyester-based resin may be crosslinked with a melamine resin or the like. Examples of the melamine resin include "SUMIMAR (registered trademark) UR" series (manufactured by Sumitomo Chemical Co., Ltd.) and "CYMEL (registered trademark)" series (manufactured by Mitsui Cytec Ltd.).

Furthermore, from the viewpoint of the balance between the workability and the durability, it is preferable that the ratio of the resin and a crosslinking agent is set so that the content of the crosslinking agent (after the reaction) to be blended in the dried first adhesive layer is in the range of 5% by mass or more and 30% by mass or less.

The resin (A) may be used alone or in combination of two or more kinds thereof.

The content of the resin (A) in the first adhesive is preferably 70% by mass or more, more preferably 80% by mass or more, still more preferably 90% by mass or more, and particularly preferably 95% by mass or more with respect to a total mass (100% by mass) of the resin contained in the first adhesive. The content of the resin (A) in the first adhesive may be 100% by mass with respect to the total mass (100% by mass) of the resins contained in the first adhesive.

The first adhesive may contain a resin other than the resin (A). Examples of such a resin include an acrylic resin, a urethane-based resin, a polyolefin-based resin, a fluorine-based resin, a silicone-based resin, and a mixture or modified resin of these resins. In addition, the first adhesive may contain additives such as a solvent (for example, cyclohexanone, or propylene glycol-1-methyl ether acetate (PGMEA)), a surfactant, and an antifoaming agent as appropriate.

The film thickness of the first adhesive layer is not particularly limited as long as the respective members can be adhered. Examples of a lower limit value of the thickness of the first adhesive layer include 5 μm or more, 10 μm or more, 15 μm or more, 20 μm or more, 25 μm or more, 30 μm or more, 35 μm or more, 40 μm or more, and 45 μm or more. Examples of the upper limit value of the thickness of the first adhesive layer include 100 μm or less, 95 μm or less, 90 μm or less, 85 μm or less, 80 μm or less, 75 μm or less, 70 μm or less, 650 μm or less, and 60 μm or less. Examples of a range of the thickness of the first adhesive layer include 5 μm to 100 μm, 10 μm to 95 μm, 15 μm to 90 μm, 20 μm to 85 μm, 25 μm to 80 μm, 30 μm to 75 μm, 35 μm to 70 μm, 40 μm to 65 μm, and 45 μm to 60 μm. The thickness provided as an exemplary above is the thickness of one first adhesive layer.

<<Second Adhesive Layer>>

The adhesive layer may include a second adhesive layer in addition to the first adhesive layer. The second adhesive layer is formed using a second adhesive. The second adhesive contains a resin having a Tg of lower than 37°C (hereinafter also referred to as a resin (B)). By using the second adhesive containing the resin (B), the adhesion of the respective members is more easily performed.

Examples of the resin (B) include a polyester-based resin having a Tg of lower than 37°C. Examples of the polyester-based resin include a polyester resin and a polyester urethane resin.

Examples of a commercially available product of the polyester resin having a Tg of lower than 37°C include VYLON 300 (7°C), VYLON 630 (7°C), VYLON 650 (10°C), VYLON GK130 (15°C), VYLON GK140 (20°C), VYLON GK150 (20°C), VYLON GK190 (11°C) VYLON GK330 (16°C), VYLON GK590 (15°C), VYLON GK680 (10°C), VYLON GK780 (36°C), VYLON GK890 (17°C), VYLON 500 (4°C), VYLON 550 (-15°C), and VYLON GK570 (0°C) (all manufactured by Toyobo Co., Ltd.), ELITEL UE-3510 (-25°C), ELITEL UE-3400 (-20°C), ELITEL UE -3220 (5°C), and ELITEL UE-3500 (15°C) (all manufactured by Unitika Ltd.), and G6 (-60°C) (manufactured by Kurabo Industries Ltd.). The numbers in parentheses indicate Tg.

Examples of the number-average molecular weight (Mn) of the polyester resin as the resin (B) include 5,000 to 100,000.

Examples of a commercial product of the polyester urethane resin having a Tg of lower than 37°C include VYLON UR-8300 (23°C), VYLON UR-3500 (10°C), and VYLON UR-6100 (-30°C). The numbers in parentheses indicate Tg.

Examples of the number-average molecular weight (Mn) of the polyester urethane resin as the resin (B) include 5,000 to 100,000.

The polyester-based resin may be crosslinked with a melamine resin or the like. Examples of the melamine resin include "SUMIMAR (registered trademark) UR" series (manufactured by Sumitomo Chemical Co., Ltd.) and "CYMEL (registered trademark)" series (manufactured by Mitsui Cytec Ltd.).

Furthermore, from the viewpoint of a balance between the workability and the durability, it is preferable that the ratio of the resin to a crosslinking agent is set so that the crosslinking agent (after the reaction) is blended to be in the range of 5% by mass or more and 30% by mass or less in the dried second adhesive layer.

The resin (B) may be a resin other than polyester-based resin. Examples of such another resin include an acrylic resin, a urethane-based resin, a polyolefin-based resin, a fluorine-based resin, a silicone-based resin, and a mixture or modified resin of these resins.

The resin (B) may be used alone or in combination of two or more kinds thereof.

The content of the resin (B) in the second adhesive is preferably 70% by mass or more, more preferably 80% by mass or more, still more preferably 90% by mass or more, and particularly preferably 95% by mass or more with respect to a total mass (100% by mass) of the resin contained in the second adhesive. The content of the resin (B) in the second adhesive may be 100% by mass with respect to the total mass (100% by mass) of the resins contained in the second adhesive.

The second adhesive may contain a resin other than the polyester-based resin having a Tg of 37°C or higher. The second adhesive may contain, for example, an acrylic resin, a urethane-based resin, a polyolefin-based resin, a fluorine-based resin, a silicone-based resin, a mixture or modified resin of these resins, or the like, each having a Tg of 37°C or higher. The second adhesive may contain additives such as a solvent (for example, cyclohexanone, or propylene glycol-1-methyl ether acetate (PGMEA)), a surfactant, and an antifoaming agent, as appropriate.

In a case where the chip for cell culture has the second adhesive layer, the thickness of the second adhesive layer is preferably 50 $\mu$m or less, more preferably 40 $\mu$m or less, still more preferably 30 $\mu$m or less, and particularly preferably 20 $\mu$m or less, or 15 $\mu$m or less. Examples of a lower limit value of the thickness of the second adhesive layer include 1 $\mu$m or more, 3 $\mu$m or more, 5 $\mu$m or more, and 7 $\mu$m or more. Examples of a range of the thickness of the second adhesive layer include 1 $\mu$m to 50 $\mu$m, 3 $\mu$m to 40 $\mu$m, 5 $\mu$m to 30 $\mu$m, 5 $\mu$m to 20 $\mu$m, 5 $\mu$m to 15 $\mu$m, and 7 $\mu$m to 15 $\mu$m. The thickness exemplified above is the thickness of one second adhesive layer. In a case where the thickness of the second adhesive layer is within the preferred range, the reduction in the drug is likely to be suppressed.

In the chip for cell culture, a proportion of a total thickness (t2) of the second adhesive layers to an overall thickness (T) of the chip for cell culture is preferably 20% or less. The total thickness (t2) of the second adhesive layers is a total value of the thicknesses of the second adhesive layers present in the chip for cell culture. By setting the proportion of the total thickness (t2) of the second adhesive layers to 20% or less of the overall thickness (T) of the chip for cell culture, the reduction in the drug is likely to be suppressed. The proportion of the thickness (t2) of the second adhesive layer to the overall thickness (T) of the chip for cell culture is preferably 17% or less, more preferably 15% or less, still more preferably 12% or less, and particularly preferably 10% or less, 9% or less, 8% or less, or 7% or less. In a case where the chip for cell culture does not have the second adhesive layer, the proportion of the thickness (t2) of the second adhesive layer is 0%.

<Configuration Example of Laminate>

The chip 1 for cell culture is composed of a laminate in which a bottom plate substrate L1, a first flow path substrate L2, a porous membrane L3, a second flow path substrate L4, a top plate substrate L5, and a cover substrate L6 are laminated in this order (hereinafter also referred to as a "laminate 100").

<<Bottom Plate Substrate L1>>

The bottom plate substrate L1 forms a bottom part of the first flow path F2. In the laminate 100, the bottom plate substrate L1 is composed of, for example, a substrate S 1. A material of the substrate S 1 is not particularly limited, but is preferably a material having high biocompatibility. From the viewpoint that observation may be performed using a phase contrast microscope or the like while culturing cells with the chip 1 for cell culture, the substrate S 1 is preferably made of a material having transparency, and more preferably made of a material with low self-fluorescence. In order to enhance the transparency, it is preferable that the substrate S 1 does not include a filler (anti-blocking agent).

Examples of the transparent material with low self-fluorescence include glass, polyethylene terephthalate (PET), polycarbonate, a cycloolefin polymer, polydimethylsiloxane, polystyrene, and polyacrylate (acryl resin). Among these, PET is preferable as the material of the substrate S 1.

The substrate S 1 may be provided with a lubricative layer containing a lubricant component on at least one surface thereof. In addition, a binder resin component may be blended into the lubricative layer.

The lubricant component is not particularly limited, and examples thereof include paraffin wax, microwax, polypropylene wax, polyethylene wax, ethylene-acrylic wax, stearic acid, behenic acid, 12-hydroxystearic acid, stearic acid amide, oleic acid amide, erucic acid amide, methylene bisstearic acid amide, ethylene bisstearic acid amide, ethylene bisoleic acid amide, butyl stearate, stearic acid monoglyceride, pentaerythritol tetrastearate, hydrogenated castor oil, stearyl stearate, siloxane, a higher alcohol-based polymer, stearyl alcohol, calcium stearate, zinc stearate, magnesium stearate, lead stearate, a silicone (dimethylsiloxane)-based low-molecular-weight substance (oil), and a silicone (dimethylsiloxane)-based resin. The lubricant component may be used alone or may be used in a combination of two or more kinds thereof.

Examples of the binder resin component to be blended in the lubricative layer include various resins such as a polyester-based resin, a polyamide-based resin, a polyurethane-based resin, an epoxy-based resin, a phenolic resin, an acrylic resin, a polyvinyl acetate-based resin, a cellulose-based resin, a styrene-based resin, and copolymer resins thereof. From the viewpoint of exhibiting an excellent lubricating property by combining the above-described lubricant component with the binder resin component, a styrene-acrylic copolymer resin is preferable as the binder resin component.

Furthermore, the term "silicone-based" indicates organosiloxanes. The properties thereof may be oily, rubbery, and resinous properties, which are correspondingly referred to as silicone oil, silicone rubber, and a silicone resin in this order. Since any of these have a water-repellent function, a lubrication function, a mold release function, and the like, in a case where the organosiloxanes are contained in the outermost layer portion of a film, the friction of the surface is effectively decreased.

The thickness of the substrate S 1 is not particularly limited, but can be, for example, 50 $\mu$m to 300 $\mu$m. Examples of the thickness of the substrate S 1 include 100 $\mu$m to 250 $\mu$m, and 150 $\mu$m to 200 $\mu$m.

The thickness of the bottom plate substrate L1 is not particularly limited, but can be, for example, 50 $\mu$m to 500 $\mu$m. Examples of the thickness of the substrate S 1 include 100 $\mu$m to 300 $\mu$m, and 150 $\mu$m to 200 $\mu$m.

<<First Flow Path Substrate L2>>

A first flow path F2 is formed in the first flow path substrate L2. In the chip 1 for cell culture, the first flow path substrate L2 forms a partition wall of the first flow path F2. In the laminate 100, the first flow path substrate L2 preferably has a multilayer structure.

The first flow path substrate L2 preferably includes the substrate S 1 and the first adhesive layer.

In the first flow path substrate L2, the layer consisting of the substrate S 1 (hereinafter also referred to as a "substrate S1 layer") may have only one layer or may have a plurality of layers. Examples of the number of substrate S 1 layers in the first flow path substrate L2 include 1 to 10, 1 to 6, 1 to 5, 1 to 4, 1 to 3, and 1 or 2. In a case where the first flow path substrate L2 includes a plurality of substrates S1, the plurality of the substrates S 1 may be the same as or different from each other. Examples of the substrate S1 include the same substrates as those for the substrate S1 in the bottom plate substrate L1.

In the first flow path substrate L2, the first adhesive layer may have only one layer or may have a plurality of layers, but preferably has two or more layers. The number of the first adhesive layers can be appropriately selected according to the number of layers of the substrate S 1. Examples of the number of the first adhesive layers on the first flow path substrate L2 include 1 to 10, 1 to 8, 1 to 6, 1 to 5, 1 to 4, 1 to 3, 2 to 10, 2 to 8, 2 to 6, 2 to 5, 2 to 4, 2 or 3, and 1 or 2. In a case where the first flow path substrate L2 includes a plurality of the first adhesive layers, the plurality of the first adhesive layers may be formed of the same first adhesive or different first adhesives.

The first flow path substrate L2 may include a second adhesive layer in addition to the substrate S1 layer and the first adhesive layer. By appropriately arranging the second adhesive layer, it is possible to enhance the adhesiveness of the respective layers within the first flow path substrate L2. In addition, the adhesiveness between the first flow path substrate L2 and the bottom plate substrate L1 and/or the porous membrane L3 can be enhanced. In a case where the first flow path substrate L2 includes a second adhesive layer, the second adhesive layer may have only one layer or may have a plurality of layers, but preferably has two or more layers. The number of the second adhesive layers can be appropriately selected according to the number of the layers of the substrate S 1 and the number of the first adhesive layers. Examples of the number of the second adhesive layers on the first flow path substrate L2 include 1 to 16, 1 to 12, 1 to 14, 1 to 12, 1 to 10, 1 to 8, 1 to 6, 1 to 5, 1 to 4, 1 to 3, 2 to 16, 2 to 12, 2 to 14, 2 to 12, 2 to 10, 2 to 8, 2 to 6, 2 to 5, 2 to 4, 2 or 3, and 1 or 2. In a case where the first flow path substrate L2 includes a plurality of the second adhesive layers, the plurality of the second adhesive layers may be formed of the same second adhesive or different second adhesives.

Specific examples of the layer configuration of the first flow path substrate L2 include a configuration in which the first flow path substrate L2 consists of a lower adhesive layer, a substrate layer, and an upper adhesive layer in this order from the bottom.

The substrate layer is a layer including the substrate S1. The substrate layer may have a monolayer structure or a multilayer structure. In a case where the substrate layer has a monolayer structure, the substrate layer consists of one layer of the substrate S1 layer. In a case where the substrate layer has a multilayer structure, the substrate layer includes two or more substrate S1 layers. In a case where the substrate layer includes two or more substrate S1 layers, the first adhesive layer and/or the second adhesive layer may be arranged between the substrate S1 layers. Examples of specific examples of the configuration of the substrate layer include a configuration in which the substrate layer includes three substrates S1, which are configured to be adhered through a second adhesive layer.

The lower adhesive layer is provided to adhere the bottom plate substrate L1 and the substrate layer to each other. The lower adhesive layer may have a monolayer structure or a multilayer structure. The lower adhesive layer may include any one of the first adhesive layer and the second adhesive layer, or may include both the first adhesive layer and the second adhesive layer. The lower adhesive layer preferably includes the first adhesive layer, and more preferably includes both the first adhesive layer and the second adhesive layer. Specific examples of the configuration of the lower adhesive layer include a configuration in which the lower adhesive layer consists of the second adhesive layer, the first adhesive layer, and the second adhesive layer in this order from the bottom.

The upper adhesive layer is provided to adhere the porous membrane L3 and the substrate layer to each other. The upper adhesive layer may have a monolayer structure or a multilayer structure. The upper adhesive layer may include any one of the first adhesive layer and the second adhesive layer or may include both the first adhesive layer and the second adhesive layer. The upper adhesive layer preferably includes the first adhesive layer, and more preferably includes both the first adhesive layer and the second adhesive layer. Specific examples of the configuration of the upper adhesive layer include a configuration in which the upper adhesive layer consists of the second adhesive layer, the first adhesive layer, and the second adhesive layer in this order from the bottom.

The thickness of the first flow path substrate L2 defines the height (the width in the vertical direction) of the first flow path F2. Therefore, the thickness of the first flow path substrate L2 can be appropriately set according to a desired height of the first flow path F2. The thickness of the first flow path substrate L2 may be appropriately adjusted, for example, by modifying the thickness and the number of the substrates S1.

Examples of the thickness of the first flow path substrate L2 include 300 $\mu$m to 2,000 $\mu$m. Examples of the thickness of the first flow path substrate L2 include 300 $\mu$m to 2,000 $\mu$m, 400 to 1,800 $\mu$m, 500 $\mu$m to 1,500 $\mu$m, 600 $\mu$m to 1,200 $\mu$m, 600 $\mu$m to 1,000 $\mu$m, 600 $\mu$m to 9,000 $\mu$m, 600 $\mu$m to 800 $\mu$m, and 700 $\mu$m to 800 $\mu$m.

<<Porous Membrane L3>>

The porous membrane L3 partitions the first flow path F2 and the second flow path F4. The porous membrane L3 is composed of a porous membrane.

The porous membrane is not particularly limited as long as it has a pore size through which cells to be cultured do not pass and a drug solution can pass. Examples of an average pore size of the porous membrane include 0.05 to 10 $\mu$m. Examples of a pore density of the porous membrane include approximately $10^5$ to $10^9$ pores/cm$^2$.

The porous membrane is preferably composed of a highly biocompatible material. Examples of the material of the porous membrane include polycarbonate, polyester, polyethylene terephthalate, and polytetrafluoroethylene.

The thickness of the porous membrane is not particularly limited. Examples of the thickness of the porous membrane include 0.1 $\mu$m to 100 $\mu$m, 0.5 $\mu$m to 50 $\mu$m, 1 $\mu$m to 30 $\mu$m, 5 $\mu$m to 20 $\mu$m, and 5 $\mu$m to 15 $\mu$m.

<<Second Flow Path Substrate L4>>

A second flow path F4 is formed in the second flow path substrate L4. In the chip for cell culture, the second flow path substrate L4 forms a partition wall of the second flow path F4. In the laminate 100, the second flow path substrate L4 preferably has a multilayer structure.

The second flow path substrate L4 preferably includes the substrate S1 and a first adhesive layer. Examples of the number of the substrate S1 layers and the first adhesive layers in the second flow path substrate L4 are the same as those exemplified for the first flow path substrate L2.

The second flow path substrate L4 may include a second adhesive layer in addition to the substrate S1 and the first adhesive layer. Examples of the number of second adhesive layers in the second flow path substrate L4 are the same as those exemplified for the first flow path substrate L2.

Specific examples of the layer configuration of the second flow path substrate L4 are the same as those exemplified for the specific examples of the first flow path substrate L2. For example, specific examples of the layer configuration of the second flow path substrate L4 include a configuration in which the second flow path substrate L4 is composed of, in the following order from the bottom, a lower adhesive layer consisting of the second adhesive layer, the first adhesive layer, and the second adhesive layer; a substrate layer consisting of the substrate S1, the second adhesive layer, the substrate S1, the second adhesive layer, and the substrate S1; and an upper adhesive layer consisting of the second adhesive layer, the first adhesive layer, and the second adhesive layer.

The lower adhesive layer is provided to adhere the porous membrane L3 and the substrate layer to each other.

The upper adhesive layer is provided to adhere the top plate substrate L5 and the substrate layer to each other.

The thickness of the second flow path substrate L4 defines a height (a width in the vertical direction) of the second flow path F4. Therefore, the thickness of the second flow path substrate L4 can be appropriately set according to the height of the target second flow path F4. The thickness of the second flow path substrate L4 may be appropriately adjusted, for example, by modifying the thickness and the number of the substrates S 1.

Examples of the thickness of the second flow path substrate L4 include 300 μm to 2,000 μm. Examples of the thickness of the first flow path substrate L2 include 300 μm to 2,000 μm, 400 to 1,800 μm, 500 μm to 1,500 μm, 600 μm to 1,200 μm, 600 μm to 1,000 μm, 600 μm to 9,000 μm, 600 μm to 800 μm, and 700 μm to 800 μm.

«Top Plate Substrate L5»

The top plate substrate L5 forms a ceiling of the second flow path F4. In the laminate 100, the top plate substrate L5 may have a monolayer structure or a multilayer structure.

In a case where the top plate substrate L5 has the monolayer structure, the top plate substrate L5 may be composed of one substrate S 1 layer. In a case where the top plate substrate L5 has the multilayer structure, it is preferable that the top plate substrate L5 includes the substrate S1 and a first adhesive layer and/or a second adhesive layer, and it is more preferable that the top plate substrate L5 includes two or more substrates S1. In a case where the top plate substrate L5 includes two or more substrate S1 layers, the first adhesive layer and/or the second adhesive layer may be arranged between the substrate S1 layers. In a case where the top plate substrate L5 includes a plurality of substrates S 1, the plurality of the substrates S 1 may be the same as or different from each other. Examples of the substrate S 1 include the same substrates as those for the substrate S1 in the bottom plate substrate L1.

Specific examples of the layer configuration of the top plate substrate L5 include a configuration in which the top plate substrate L5 consists of the substrate S 1 layer, the second adhesive layer, and the substrate S1 layer in this order from the bottom. In the specific example, the top plate substrate L5 includes two substrates S1, which are configured to be adhered through the second adhesive layer. The top plate substrate L5 may be the one consisting of one layer of the substrate S 1 layer.

The thickness of the top plate substrate L5 is not particularly limited, but can be, for example, 50 μm to 600 μm. Examples of the thickness of the top plate substrate L5 include 100 μm to 500 μm, and 150 μm to 400 μm.

<<Cover Substrate L6>>

The cover substrate L6 is provided on the uppermost layer of the chip for cell culture. In the laminate 100, the cover substrate L6 may have a monolayer structure or a multilayer structure.

In a case where the uppermost layer of the top plate substrate L5 is the substrate S 1 layer, the lowermost layer of the cover substrate L6 is preferably the first adhesive layer or the second adhesive layer. In a case where the uppermost layer of the top plate substrate L5 is the first adhesive layer or the second adhesive layer, the lowermost layer of the cover substrate L6 may be the substrate S 1 layer or a layer consisting of the substrate S2 (hereinafter also referred to as the "substrate S2 layer").

Specific examples of the configuration of the cover substrate L6 include a configuration in which the cover substrate L6 consists of, in the following order from the bottom, an adhesive layer consisting of the second adhesive layer, the first adhesive layer, and the second adhesive layer; and a substrate S2.

From the viewpoint that observation may be performed using a phase contrast microscope or the like while culturing cells with the chip 1 for cell culture, the substrate S2 is preferably made of a material having transparency, and more preferably made of a material with low self-fluorescence. In order to enhance the transparency, it is preferable that the substrate S2 does not include a filler (anti-blocking agent).

Examples of the transparent material with low self-fluorescence include glass, polyethylene terephthalate (PET), polycarbonate, a cycloolefin polymer, polydimethylsiloxane, polystyrene, polyacrylate (acryl resin), and polymethyl methacrylate (PMMA). Among these, PMMA is preferable as the material of the substrate S2.

The thickness of the substrate S2 is not particularly limited, but can be, for example, 500 μm to 5,000 μm. Examples of the thickness of the substrate S2 include 800 μm to 3,000 μm, 1,000 μm to 2,500 μm, and 1,500 μm to 2,500 μm.

The thickness of the cover substrate L6 is not particularly limited, but can be, for example, 500 μm to 5,000

μm. Examples of the thickness of the substrate S2 include 800 μm to 3,000 μm, 1,000 μm to 2,500 μm, and 1,500 μm to 2,500 μm.

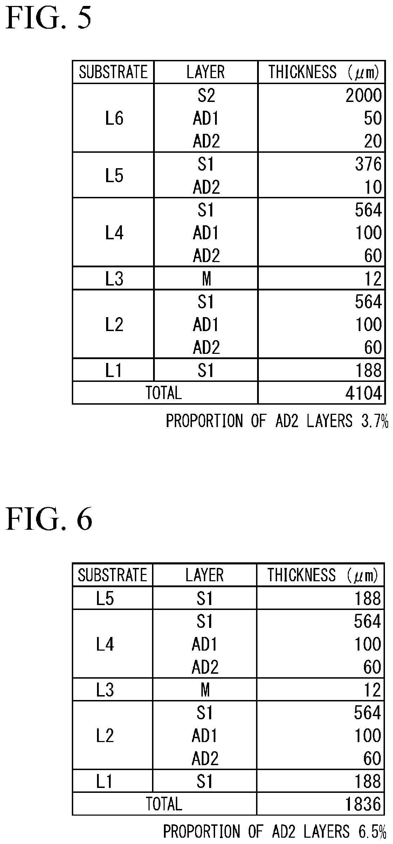

Specific examples of the thickness occupied by each layer in each substrate of the laminate 100 are shown in FIG. 5. In FIG. 5, L1 represents the bottom plate substrate L1, L2 represents the first flow path substrate L2, L3 represents the porous membrane L3, L4 represents the second flow path substrate L4, L5 represents the top plate substrate L5, and L6 represents the cover substrate L6. S1 represents the substrate S1 layer and S2 represents the substrate S2 layer. AD1 represents a first adhesive layer and AD2 represents a second adhesive layer. M represents a porous membrane.

In FIG. 5, the thickness of each layer indicates a total value of the thicknesses of the respective layers in the respective substrates. For example, in FIG. 5, the thickness "60 μm" of the layer "AD2" of the substrate "L2" is a total value of the thicknesses of the second adhesive layers in the first flow path substrate L2. In the example in FIG. 5, the thickness of the entire laminate 100 is 4,104 μm. In the entire laminate 100, the total thickness of the second adhesive layers (AD2 layers) is 150 μm. Therefore, the proportion of the total thickness (t2) of the second adhesive layers to the overall thickness (T) of the chip for cell culture is about 3.7% according to Expression (1).

$$\text{Proportion (\%) of thickness of second adhesive layer} = t2/T \times 100 \qquad (1)$$

A plurality of the substrates S 1 included in the laminate 100 may all be composed of the same material, or may be partially composed of different materials. The thicknesses of the plurality of the substrates S 1 may be the same as or different from each other.

The plurality of the first adhesive layers included in the laminate 100 may all be composed of the same first adhesive, or may be partially composed of different first adhesives. The thicknesses of the plurality of the first adhesive layers may be the same as or different from each other.

The plurality of the second adhesive layers included in the laminate 100 may all be composed of the same second adhesive, or may be partially composed of different second adhesives. The thicknesses of the plurality of the second adhesive layers may be the same as or different from each other.

<Other Configuration Examples of Laminate>

The chip for cell culture may not include the cover substrate L6. For example, the chip for cell culture may be composed of a laminate in which the bottom plate substrate L1, the first flow path substrate L2, the porous membrane L3, the second flow path substrate L4, and the top plate substrate L5 are laminated in this order (hereinafter referred to as a "laminate 200").

In the laminate 200, examples of the bottom plate substrate L1, the first flow path substrate L2, the porous membrane L3, the second flow path substrate L4, and the top plate substrate L5 are the same as those provided as exemplary examples in the laminate 100.

In the laminate 200, specific examples of the top plate substrate L5 include those composed of one layer of the substrate S 1 layer.

In the laminate 200, the thickness of the top plate substrate L5 is not particularly limited, and examples thereof include 50 μm to 300 μm, 100 μm to 200 μm, and 150 μm to 200 μm.

A specific example of the thickness occupied by each layer in each substrate of the laminate 200 is shown in FIG. 6. In FIG. 6, L1 represents the bottom plate substrate L1, L2 represents the first flow path substrate L2, L3 represents the porous membrane L3, L4 represents the second flow path substrate L4, and L5 represents the top plate substrate L5. S1 represents the substrate S1 layer. AD1 represents a first adhesive layer and AD2 represents a second adhesive layer. M represents a porous membrane.

In FIG. 6, the thickness of each layer indicates a total value of the thicknesses of the respective layers in each substrate. For example, in FIG. 6, the thickness "60 μm" of the layer "AD2" of the substrate "L2" is a total value of the thicknesses of the second adhesive layers in the first flow path substrate L2. In the example in FIG. 6, the thickness of the entire laminate 200 is 1,836 μm. In the entire laminate 200, the total thickness of the second adhesive layers (AD2 layers) is 120 μm. Therefore, the proportion of the total thickness (t2) of the second adhesive layers to the overall thickness (T) of the chip for cell culture is about 6.5% according to Expression (1).

The relationship among the plurality of the substrates S 1, the plurality of the first adhesive layers AD1, and the plurality of the second adhesive layers AD2 included in the laminate 200 is the same as that in the laminate 100.

(Modification Examples)

The chip for cell culture may further have a flow path substrate in addition to the first flow path substrate L2 and the second flow path substrate L4. In addition, a bottom plate substrate for the additional flow path, a porous membrane, and a top plate substrate may be provided. The number of the flow path substrates to be added is not particularly limited, and can be set to any number.

According to the chip for cell culture of the present embodiment, a first adhesive layer formed of a first adhesive that contains a polyester-based resin having a Tg of 37°C or higher and 120°C or lower is included as an adhesive layer for adhering the respective layers of the laminate constituting the chip for cell culture. As a result, in a case where a drug solution is perfused or retained in the flow path, the sorption of the drug onto the adhesive layer exposed to the flow path is suppressed. As a result, a reduction in the drug in the drug solution is suppressed. Accordingly, it is possible to obtain a chip for cell culture in which the reduction in the drug is suppressed.

Since the chip for cell culture of the present embodiment suppresses a reduction in the drug in a case where the drug solution is perfused or retained in the flow path, the chip for cell culture can be suitably used for cell culture in the presence of the drug. The chip for cell culture of the present embodiment can be applied to, for example, an evaluation test (an evaluation test for an efficacy, an evaluation test for a safety, and the like) for a drug using cells.

(Device for Cell Culture)

A second aspect of the present invention is a device for cell culture, including the chip for cell culture of the first aspect.

A device for cell culture is provided with the chip for cell culture of the first aspect, and a mechanism for performing cell culture in the chip for cell culture. The mechanism provided in the device for cell culture can be appropriately modified depending on the purpose of cell culture.

Examples of the mechanism provided in the device for cell culture include a liquid feeding mechanism for supplying a drug solution to a flow path of a chip for cell culture (a drug solution tank, a liquid feeding tube, a delivery pump, or the like); a drainage mechanism for discharging a drug solution from a flow path of a chip for cell culture (a drainage tank, a drainage tube, a discharge pump, or the like); a temperature maintenance mechanism for maintaining a culture temperature of a chip for cell culture (a thermostat or the like); and a washing mechanism for washing a flow path of a chip for cell culture (a washing liquid tank, a tube, a pump, or the like).

Since the device for cell culture of the present aspect includes the chip for cell culture of the first aspect, cell culture can be performed while suppressing a reduction in a drug in a drug solution. Therefore, the device for cell culture can be suitably used for cell culture in the presence of the drug. The device for cell culture of the present aspect can be applied to, for example, an evaluation test (an evaluation test for an efficacy, an evaluation test for a safety, and the like) for a drug using cells.

(Method for Producing Chip for Cell Culture)

According to a third aspect of the present invention, there is provided a method for producing a chip for cell culture, including a laminate having a flow path structure inside. The production method according to the present aspect includes a step A of laminating the respective members constituting the respective layers of the laminate through adhesive layers; and a step B of adhering the respective laminated members by the adhesive layers. The adhesive layer includes a first adhesive layer formed of an adhesive that contains a polyester-based resin having a glass transition temperature of 37°C or higher and 120°C or lower.

<Step A>

The step A is a step of laminating the respective members constituting the respective layers of the laminate through adhesive layers.

Examples of the respective members of the laminate constituting the chip for cell culture include the bottom plate substrate L1, the first flow path substrate L2, the porous membrane L3, the second flow path substrate L4, the top plate substrate L5, and the cover substrate L6.

<<Bottom Plate Substrate L1>>

As the bottom plate substrate L1, the same substrate as described above can be used. The bottom plate substrate L1 can be manufactured by processing the substrate S1 into a size of a chip for cell culture, by laser processing or the like. For example, a carbon dioxide laser or the like can be used for the laser processing.

**<<First Flow Path Substrate L2>>**

As the first flow path substrate L2, the same substrate as described above can be used. The first flow path substrate L2 preferably has a multilayer structure. In a case where the first flow path substrate L2 has a multilayer structure, it is possible to obtain a laminate for the first flow path substrate L2 by laminating the respective layers constituting the first flow path substrate L2 through the adhesive layers, followed by compression-bonding. The compression-bonding of the respective layers can be performed using a laminator, a press machine, or the like. For example, a laminate for the first flow path substrate L2 in which the respective layers are adhered through the adhesive layers can be obtained by laminating the respective layers, followed by compression-bonding by a laminator, and then pressing by a press machine.

In a case where the first flow path substrate L2 includes a plurality of the substrates S1, for example, the first flow path substrate L2 can be manufactured as follows. First, the first adhesive or the second adhesive is applied to one surface or both surfaces of the substrate S 1. It is possible to apply the first adhesive or the second adhesive onto the substrate S1 using a bar coater or the like. After applying the first adhesive or the second adhesive, it is preferable to remove a solvent component by using an oven or the like. Next, any number of the substrates S 1 having the adhesive applied thereon are laminated and compression-bonded, whereby it is possible to obtain a substrate layer to which the substrate S1 is adhered by the first adhesive layer or the second adhesive layer.

Next, the first adhesive or the second adhesive is applied to both surfaces of the obtained substrate layer. In a case where a plurality of the adhesive layers are formed, the first adhesive layer may be applied and dried, and then the second adhesive layer may be applied thereon and dried. By repeating this step, a desired number of adhesive layers can be formed.

In this manner, a laminate for the first flow path substrate L2 can be obtained.

Alternatively, sheets for forming the respective layers of the first flow path substrate L2 may be individually prepared, and these sheets may be laminated in a desired order and compression-bonded, thereby manufacturing the first flow path substrate L2. For example, a desired number of each of the substrate S 1, the first adhesive sheet for forming the first adhesive layer, and the second adhesive sheet for forming the second adhesive layer is prepared. Next, the substrate S1, the first adhesive sheet, and the second adhesive sheet are laminated in a desired order to form a laminate. In a case where a plurality of the substrates S 1 are used, it is preferable that the first adhesive sheet and/or the second adhesive sheet is interposed between the two substrates S1. The lowermost layer and the uppermost layer of the laminate are preferably the first adhesive sheet or the second adhesive sheet. Next, the respective layers of the laminate are compression-bonded using a laminator, a press machine, or the like.

In this manner, a laminate for the first flow path substrate L2 can be obtained.

For example, in a case where the first flow path substrate L2 includes three substrate S1 layers, a laminate for the first flow path substrate L2 may be obtained as follows. First, three substrates S1, two first adhesive sheets, and six second adhesive sheets are prepared. Next, for example, the second adhesive sheet, the first adhesive sheet, the second adhesive sheet, the substrate S1, the second adhesive sheet, the substrate S1, the second adhesive sheet, the substrate S1, the second adhesive sheet, the first adhesive sheet, and the second adhesive sheet are laminated in this order from the bottom to form a laminate. Next, the respective layers of the laminate are compression-bonded using a laminator, a press machine, or the like.

In this manner, a laminate for the first flow path substrate L2 including three substrate S1 layers can be obtained.

The laminate for the first flow path substrate L2 can be processed into the size of the chip for cell culture by laser processing or the like. Furthermore, a flow path is formed by laser processing or the like. For example, a carbon dioxide laser or the like can be used for the laser processing.

**<<Porous Membrane L3>>**

As the porous membrane L3, the same membrane as described above can be used. The porous membrane L3 can be processed into the size of the chip for cell culture by laser processing or the like. Furthermore, necessary processing such as formation of through holes for the port P1 and the port P2 is performed by laser processing or the like.

**<<Second Flow Path Substrate L4>>**

As the second flow path substrate L4, the same substrate as described above can be used. The second flow path substrate L4 preferably has a multilayer structure. The second flow path substrate L4 can be manufactured by the same method as the first flow path substrate L2.

«Top Plate Substrate L5»

As the top plate substrate L5, the same substrate as described above can be used. The top plate substrate L5 may have a multilayer structure or a monolayer structure. In a case where the top plate substrate L5 has the multilayer structure, a laminate for the top plate substrate L5 can be manufactured by the same method as for the first flow path substrate L2.

The laminate for the top plate substrate L5 or the substrate for the top plate substrate L5 can be processed into the size of the chip for cell culture by laser processing or the like. Furthermore, the top plate substrate L5 can be manufactured by performing necessary processing such as formation of openings for the ports P1 to P4 by laser processing or the like.

<<Cover Substrate L6>>

As the cover substrate L6, the same substrate as described above can be used. The cover substrate L6 can be processed into the size of the chip for cell culture by laser processing or the like. Furthermore, the top plate substrate L5 can be manufactured by performing necessary processing such as formation of through holes for the ports P1 to P4 and an opening part W by laser processing or the like.

For the respective members prepared as described above, the bottom plate substrate L1, the first flow path substrate L2, the porous membrane L3, the second flow path substrate L4, the top plate substrate L5, and the cover substrate L6 are laminated in this order. At the time of laminating the respective members, the members are laminated so that the positions of the through holes for the ports P1 to P4 are aligned. Furthermore, in a case where the chip for cell culture does not include the cover substrate L6, lamination of the cover substrate L6 is not performed.

<Step B>

The step B is a step of adhering the respective laminated members by the adhesive layers.

The adhesion of the respective members can be performed using a laminator, a press machine, or the like. For example, by compression-bonding the respective members of the laminate with a laminator, followed by pressing with a press machine, it is possible to obtain a chip for cell culture, in which the respective members are adhered through the adhesive layers.

The chip for cell culture produced by the production method of the present embodiment is the chip for cell culture of the first aspect. Therefore, the production method according to the present embodiment can be applied to production of the chip for cell culture of the first aspect.

(Method for Culturing Cells)

A fourth aspect of the present invention is a method for culturing cells. The culture method according to the present aspect includes a step of culturing cells in the presence of a drug within the flow path of the chip for cell culture of the first aspect.

<Culturing Step>

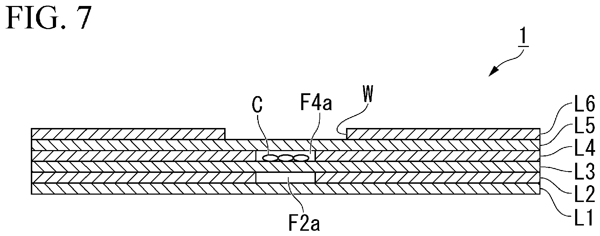

FIG. 7 is a schematic view showing an example of a culture state in the present step. In the chip 1 for cell culture, the first central flow path F2a and the second central flow path F4a are partitioned by the porous membrane L3. Cells C are cultured on the porous membrane L3 facing the second central flow path F4a.

The cells C are not particularly limited and any cell can be used. Animal cells can be used as the cells C. Examples of the animal cells include human cells, and cells of non-human animals (monkeys, mice, rats, guinea pigs, marmosets, dogs, cats, insects, and the like).

The kind of the cells is not particularly limited, and can be appropriately selected according to the intended purpose. Examples of the cells include, but are not limited to, immune cells, germ cells, nerve cells, fibroblasts, mesenchymal stem cells, hormone-secretory cells, cells of various organs, cancer cells, cells of various diseases, and pluripotent stem cells.

Any drug can be used. The drug may be a candidate drug to be developed as a therapeutic agent for any disease. Examples of the drug include, but are not limited to, a low-molecular-weight drug (a molecular weight of 500 or less), a medium-molecular-weight drug (a molecular weight of about 500 to 2,000), and a high-molecular-weight drug (a nucleic acid drug, a protein drug, a polymer, or the like).

The medium that is used in the culture is not particularly limited, and can be appropriately selected according to the kind of the cell. The medium may be a basal medium for animal culture, to which a drug for evaluation and necessary components have been added as appropriate. A known basal medium for animals can be used.

Examples of the basal medium include a Doulbecco's modified Eagle's medium (DMEM), a DMEM/F12 medium, an IMDM medium, a Medium199 medium, an Eagle's minimum essential medium (EMEM), an αMEM medium, a Ham's F12 medium, an RPMI 1640 medium, a Fischer's medium, and a mixed medium thereof.

The basal medium may include a serum (such as a fetal bovine serum (FBS)) or a serum replacement, as necessary. Examples of the serum replacement include albumin, transferrin, sodium selenite, ITS-X (Invitrogen), a knockout serum replacement (KSR), an N2 supplement (Invitrogen), a B27 supplement (Invitrogen), a fatty acid, insulin, a collagen precursor, a trace element, 2-mercaptoethanol, and 3'-thiol glycerol. The basal medium may include components such as a lipid, an amino acid, L-glutamine, Glutamax, a non-essential amino acid, a vitamin, a growth factor, an antibiotic, an antioxidant, pyruvic acid, a buffer, and inorganic salts, as necessary. These can be appropriately used in combination.

As the culture conditions, conditions usually used for culturing animal cells can be used. The culture temperature can be, for example, 32°C to 40°C, and is preferably 35°C to 38°C (for example, 37°C). The $CO_2$ concentration can be, for example, about 2% to 5% (for example, 5%).

A culture procedure can be performed, for example, as follows.

First, a medium is injected from the port P1 and introduced into the first central flow path F2a. Next, a cell culture solution is injected from the port P3 and introduced into the second central flow path F4a. Incubation is performed for any time while the cell culture solution is retained in the second central flow path F4a, thereby allowing the cells to be fixed on the porous membrane L3. Next, the drug solution is injected from the port P3 and introduced into the second central flow path F4a, and cell culture is performed. During the cell fixation and the culture, the drug solution may be perfused through the second flow path F4 or may be retained within the second flow path F4. During the culture, the medium may be perfused through the first flow path F2 or may be retained within the first flow path F2.

It is possible to evaluate an effect of the drug on the cells by analyzing the state of the cells during or after the culture.

In the culture method of the present embodiment, since the culture is performed using the chip for cell culture according to the first aspect, it is possible to suppress the reduction in the drug due to sorption of the chip for cell culture. Therefore, an effect of the drug on cells can be accurately evaluated.

[Examples]

Hereinafter, the present invention will be described in more detail with reference to Examples, but the present invention is not limited thereto.

(Test on Reduction in Drug)

<Manufacture of Disc Sample with Adhesive Layer>

Each adhesive shown in Table 1 was evaluated. In a case where the adhesive was a solid feed, the adhesive was dissolved in any solvent and then used. The adhesive solution was applied onto an easily adhesive surface of a PET film (thickness 188 μm, Cosmoshine A4160, manufactured by Toyobo Co., Ltd.) with a bar coater so that the film thickness after drying was 10 μm. Next, the PET film was dried in an oven at 100°C for 3 hours to remove the solvent, thereby obtaining a PET film with an adhesive layer. Next, using a carbon dioxide laser (speed: 1 to 80 m/s, Power: 15 to 95; Spirit 60w S-60, manufactured by Comnet Inc.), the PET film with the adhesive layer was processed into a disc shape having a diameter of 10 mm, thereby obtaining a disc sample with an adhesive layer.

[Table 1]

|  | Name of Product | Composition | Name of Manufacturer | Glass transition temperature (°C) |
|---|---|---|---|---|
| Example 1 | VYLON UR-1350 | Polyester urethane resin | Toyobo Co., Ltd. | 46 |
| Example 2 | VYLON 245 | Polyester resin | Toyobo Co., Ltd. | 60 |
| Example 3 | ELITEL UE-9200 | Polyester resin | Unitika Ltd | 65 |
| Example 4 | VYLON 200 | Polyester resin | Toyobo Co., Ltd. | 67 |

(continued)

|  | Name of Product | Composition | Name of Manufacturer | Glass transition temperature (°C) |
|---|---|---|---|---|
| Example 5 | KuranBeter G7 | Polyester resin | Kurabo Industries Ltd. | 78 |
| Comparative Example 1 | KuranBeter G6 | Polyester resin | Kurabo Industries Ltd. | -60 |
| Comparative Example 2 | VYLON UR-8700 | Polyester urethane resin | Toyobo Co., Ltd. | -22 |
| Comparative Example 3 | VYLON 500 | Polyester resin | Toyobo Co., Ltd. | 4 |
| Comparative Example 4 | VYLON GK780 | Polyester resin | Toyobo Co., Ltd. | 36 |

<Method for Test on Reduction in Drug>

Using a disc sample with the adhesive layer manufactured above, a test on a reduction in five kinds of drugs shown below was performed.

PHN: Phenacetin

DIC: Diclofenac

MEP: (S)-Mephenytoin

BUF: Bufuralol

MDZ: Midazolam

The disc sample with the adhesive layer manufactured above was sterilized by being immersed in 70% ethanol for 30 minutes, and air-dried in a clean bench. Next, 50 $\mu$L of the drug solution was introduced onto a bottom surface of a well of a 24-well plate. Next, the disc sample with the adhesive layer was suspended on the drug solution so that the surface of the adhesive layer was on the drug solution side, and left to stand in an incubator at 37°C for 48 hours. Thereafter, the disc sample with the adhesive layer was removed from the well. 150 $\mu$L of a diluent solvent for LC-MS/MS measurement was introduced into the well, and the entire amount was recovered together with the drug solution. The concentration of the drug in the recovered sample was quantified by LC-MS/MS (EXION AD-QTRAP6500+, manufactured by SCIEX). As a relative value in a case where the concentration of the drug in the drug solution before the contact of the disc sample with adhesive layer was taken as 100%, a residual ratio of the drug after 48-hour incubation was calculated and is shown in Table 2 as "Residual ratio of drug". In addition, the residual ratio of the drug was comprehensively evaluated according to the following criteria, and the results are shown in Table 2 as "Comprehensive evaluation".

«Evaluation Standard»

A: The residual ratio of the drug is 60% or more with all 5 kinds of drugs.
B: The residual ratio of the drug is less than 60% with one or more kinds of drugs.

[Table 2]

| | Residual ratio (%) of drug | | | | | Comprehensive evaluation |
|---|---|---|---|---|---|---|
| | PHN | DIC | MEP | BUF | MDZ | |
| Example 1 | 96.3 | 88.2 | 89.1 | 75.1 | 83.7 | A |
| Example 2 | 100.0 | 92.9 | 92.0 | 64.3 | 82.1 | A |
| Example 3 | 85.2 | 92.5 | 91.7 | 81.9 | 90.4 | A |
| Example 4 | 100.1 | 96.0 | 95.4 | 83.7 | 89.5 | A |
| Example 5 | 101.1 | 101.6 | 103.9 | 95.3 | 100.3 | A |
| Comparative Example 1 | 57.1 | 91.2 | 31.7 | 13.6 | 33.5 | B |
| Comparative Example 2 | 84.2 | 119.3 | 63.0 | 11.0 | 49.0 | B |
| Comparative Example 3 | 104.9 | 130.9 | 83.8 | 42.0 | 59.6 | B |
| Comparative Example 4 | 79.9 | 90.5 | 79.7 | 42.7 | 65.6 | B |

From the results, it was confirmed that the adhesives of Examples can suppress a reduction in the drug, as compared with the adhesives of Comparative Examples.

(Test on Reduction in Drug Using Chip for Cell Culture)

(Manufacture of Chip for Cell Culture)

A chip for cell culture, consisting of a bottom plate substrate L1, a first flow path substrate L2, a porous membrane L3, a second flow path substrate L4, and a top plate substrate L5, was manufactured. Each of the bottom plate substrate L1 and the top plate substrate L5 consists of one substrate S 1 layer. The first flow path substrate L2 and the second flow path substrate L4 each consist of a laminate in which the second adhesive layer, the first adhesive layer, the second adhesive layer, the substrate S1 layer, the second adhesive layer, the substrate S 1 layer, the second adhesive layer, the substrate S 1 layer, the second adhesive layer, the first adhesive layer, and the second adhesive layer are laminated in this order. A material shown in Table 3 was used as the adhesive for each of the first adhesive layer and the second adhesive layer. A PET film (thickness 188 $\mu$m, Cosmoshine A4160, manufactured by Toyobo Co., Ltd.) was used as the substrate S1. As the porous membrane, a polyethylene terephthalate-made porous membrane (average pore size: 0.45 $\mu$m, ipCELLCULTURE, manufactured by it4ip S. A.) was used. The thicknesses of the respective layer in the respective substrates were as shown in FIG. 6.

[Table 3]

| | AD1 | | AD2 | |
|---|---|---|---|---|
| | Adhesive | Glass transition temperature (°C) | Adhesive | Glass transition temperature (°C) |
| Example 6 | KuranBeter G7 | 78 | VYLON 500 | 4 |
| Example 7 | KuranBeter G7 | 78 | VYLON UR-1350 | 46 |
| Example 8 | KuranBeter G7 | 78 | ELITEL UE-9200 | 35 |
| Comparative Example 5 | KuranBeter G6 | -60 | VYLON 500 | 4 |

Sheets for the respective layers of the respective members were manufactured, laminated in a desired order, and thermocompression-bonded through a laminate roll (laminator FA-570, manufactured by Taisei Laminator Co., Ltd.) set at 140°C (roll load: 0.3 MPa, speed: 0.1 to 0.5 m/min) to manufacture each of laminates for the first flow path substrate L2 and the second flow path substrate L4. This was cut out to a size of 200 mm × 150 mm. Furthermore, the respective layers of the laminate were adhered by pressing at 105°C under a load of 3 t using a small-size press machine (H300-05, manufactured by AS ONE Corporation).

The necessary laser processing was performed for the respective members using LaserPro (carbon dioxide laser, manufactured by GCC). By laser processing, the first flow path substrate L2 was formed of the first flow path F2 and the second flow path substrate L4 was formed of the second flow path F4. Through holes for the port P1 to the port P4 were formed in the top plate substrate L5. Through holes for the port P1 and the port P2 were formed in the porous membrane L3.

The bottom plate substrate L1, the first flow path substrate L2, the porous membrane L3, the second flow path substrate L4, and the top plate substrate L5 are laminated in this order, and thermocompression-bonded through a laminate roll (Laminator FA-570, manufactured by Taisei Laminator Co., Ltd.) set at 140°C to obtain a laminate for a chip for cell culture. In a case where the respective substrates were laminated, positions of each port and flow path were aligned. Furthermore, the respective layers of the laminate were adhered by pressing at 50°C to 80°C under a load of 3 t using a small-size press machine (H300-05, manufactured by AS ONE Corporation).

Then, the resultant was cut out to a size of 58 mm × 46 mm using LaserPro to obtain a chip for cell culture.

<Method for Test on Reduction in Drug>

Using the chip for cell culture manufactured above, a test on a reduction in a drug shown below was performed.

PHN: Phenacetin
DIC: Diclofenac
MEP: (S)-Mephenytoin

The inside of the flow path of the chip for cell culture manufactured above was washed with 9 mL of PBS. Next, the substrate was sterilized by irradiation with UV in a clean bench. UV irradiation was performed for 20 minutes on the front side and the back side of the chip for cell culture. Next, the inside of the flow path was washed with 6 mL of PBS and air-dried in the clean bench.

A drug solution in an amount adjusted to match the volume of the flow path is introduced into the flow path (the first flow path F2, the second flow path F4) of the chip for cell culture, and the chip for cell culture was maintained horizontally so that no air remained within the flow path. 50 μL of the drug solution was recovered immediately after the introduction of the drug solution, and mixed with 150 μL of a diluent solvent for LC-MS/MS measurement, and the mixture was used as a sample after 0-hour incubation.

50 μL of the drug solution was newly introduced into the flow path (the first flow path F2, the second flow path F4) of the chip for cell culture, and left to stand in the incubator at 37°C. After 48 hours from the introduction of the drug solution, 50 μL of the drug solution was recovered and mixed with 150 μL of a diluent solvent for LC-MS/MS measurement, and the mixture was used as a sample after 48-hour incubation.

The concentration of the drug in the sample was quantified by LC-MS/MS. As a relative value in a case where the concentration of the drug of the sample after 0-hour incubation was taken as 100%, the residual ratio of the drug of the sample after 48-hour incubation was calculated and is shown in Table 4 as "Residual ratio of drug".

[Table 4]

|  | Residual ratio (%) of drug | | |
|---|---|---|---|
|  | PHN | DIC | MEP |
| Example 6 | 73.6 | 85.3 | 33.4 |
| Example 7 | 99.5 | 107.3 | 60.4 |
| Example 8 | 92.6 | 97.7 | 99.9 |
| Comparative Example 5 | 49.3 | 76.9 | 91.3 |

It was confirmed that the chips for cell culture of Examples can suppress a reduction in the drug, as compared with the chips for cell culture of Comparative Examples.

Although preferred Examples of the present invention have been described above, the present invention is not limited to these Examples. Addition, omission, replacement, and other modifications can be made to the configuration without departing from the spirit of the present invention. The present invention is not limited by the descriptions above, but only by the scope of the accompanying claims.

[Reference Signs List]

L1: Bottom plate substrate
L2: First flow path substrate
L3: Porous membrane
L4: Second flow path substrate
L5: Top plate substrate
L6: Cover substrate
P1 to P4: Port
F2: First flow path
F4: Second flow path
W: Opening part

**Claims**

1. A chip for cell culture, comprising a laminate having a flow path structure inside,

   wherein respective members constituting respective layers of the laminate are adhered by adhesive layers, and the adhesive layer comprises a first adhesive layer formed of an adhesive that contains a polyester-based resin having a glass transition temperature of 37°C or higher and 120°C or lower.

2. The chip for cell culture according to Claim 1,
   wherein the polyester-based resin is a polyester resin or a polyester urethane resin.

3. The chip for cell culture according to Claim 1 or 2,

   wherein the adhesive layer comprises a second adhesive layer formed of an adhesive that contains a resin having a glass transition temperature of lower than 37°C, and
   a proportion of a total thickness (t2) of the second adhesive layers to an overall thickness (T) of the chip for cell culture is 20% or less.

4. The chip for cell culture according to Claim 1 or 2,
   wherein the laminate comprises a bottom plate substrate, a first flow path substrate in which a first flow path is formed, a porous membrane, a second flow path substrate in which a second flow path is formed, and a top plate substrate in this order.

5. The chip for cell culture according to Claim 4,
   wherein at least one selected from the group consisting of the first flow path substrate and the second flow path substrate has a multilayer structure.

6. A device for cell culture, comprising the chip for cell culture according to Claim 1 or 2.

7. A method for producing a chip for cell culture, comprising a laminate having a flow path structure inside, the method comprising:

   a step A of laminating respective members constituting respective layers of the laminate through adhesive layers; and
   a step B of adhering the respective laminated members by the adhesive layers,
   wherein the adhesive layer comprises a first adhesive layer formed of an adhesive that contains a polyester-based resin having a glass transition temperature of 37°C or higher and 120°C or lower.

8. The method for producing a chip for cell culture according to Claim 7,
   wherein the polyester-based resin is a polyester resin or a polyester urethane resin.

9. The method for producing a chip for cell culture according to Claim 7 or 8,

wherein the adhesive layer comprises a second adhesive layer formed of an adhesive that contains a resin having a glass transition temperature of lower than 37°C, and
a proportion of a total thickness (t2) of the second adhesive layers to an overall thickness (T) of the chip for cell culture is 20% or less.

10. The method for producing a chip for cell culture according to Claim 7 or 8,
    wherein the laminate comprises a bottom plate substrate, a first flow path substrate in which a first flow path is formed, a porous membrane, a second flow path substrate in which a second flow path is formed, and a top plate substrate in this order.

11. A method for culturing cells, comprising a step of culturing cells in the presence of a drug within the flow path of the chip for cell culture according to Claim 1 or 2.

FIG. 1

FIG. 2

FIG. 3

FIG. 4

# FIG. 5

| SUBSTRATE | LAYER | THICKNESS ($\mu$m) |
|---|---|---|
| L6 | S2 | 2000 |
| | AD1 | 50 |
| | AD2 | 20 |
| L5 | S1 | 376 |
| | AD2 | 10 |
| L4 | S1 | 564 |
| | AD1 | 100 |
| | AD2 | 60 |
| L3 | M | 12 |
| L2 | S1 | 564 |
| | AD1 | 100 |
| | AD2 | 60 |
| L1 | S1 | 188 |
| TOTAL | | 4104 |

PROPORTION OF AD2 LAYERS 3.7%

# FIG. 6

| SUBSTRATE | LAYER | THICKNESS ($\mu$m) |
|---|---|---|
| L5 | S1 | 188 |
| L4 | S1 | 564 |
| | AD1 | 100 |
| | AD2 | 60 |
| L3 | M | 12 |
| L2 | S1 | 564 |
| | AD1 | 100 |
| | AD2 | 60 |
| L1 | S1 | 188 |
| TOTAL | | 1836 |

PROPORTION OF AD2 LAYERS 6.5%

FIG. 7

# EP 4 394 025 A1

| INTERNATIONAL SEARCH REPORT | International application No. |
|---|---|
| | **PCT/JP2022/032043** |

**A. CLASSIFICATION OF SUBJECT MATTER**

*C12M 1/00*(2006.01)i; *B32B 7/12*(2006.01)i; *B32B 27/00*(2006.01)i; *B32B 27/36*(2006.01)i; *C09J 7/35*(2018.01)i; *C09J 167/00*(2006.01)i; *C12N 1/00*(2006.01)i; *C12N 5/00*(2006.01)i
FI:   C12M1/00 C; C12N1/00 A; B32B7/12; B32B27/00 C; B32B27/36; C09J7/35; C09J167/00; C12N5/00

According to International Patent Classification (IPC) or to both national classification and IPC

**B. FIELDS SEARCHED**

Minimum documentation searched (classification system followed by classification symbols)

C12M1/00; B32B7/12; B32B27/00; B32B27/36; C09J7/35; C09J167/00; C12N1/00; C12N5/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2021-114910 A (TOKYO OHKA KOGYO CO LTD) 10 August 2021 (2021-08-10) claims, paragraphs [0039]-[0097], [0113], fig. 1-7 | 1-3, 6-9, 11 |
| Y | | 4, 5, 10 |
| Y | WO 2019/168118 A1 (FUJIFILM CORPORATION) 06 September 2019 (2019-09-06) claims, examples, fig. 1-11B | 4, 5, 10 |
| Y | JP 2020-188723 A (UNIV TOHOKU) 26 November 2020 (2020-11-26) claims, examples, fig. 2-29 | 4, 5, 10 |
| A | JP 2018-102236 A (TOKYO OHKA KOGYO CO LTD) 05 July 2018 (2018-07-05) entire text | 1-11 |
| A | WO 2015/159821 A1 (TOKYO OHKA KOGYO CO LTD) 22 October 2015 (2015-10-22) paragraph [0053] | 1-11 |

☐ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 October 2022** | **25 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/032043**

| Patent document cited in search report | | | Publication date (day/month/year) | Patent family member(s) | | | Publication date (day/month/year) |
|---|---|---|---|---|---|---|---|
| JP | 2021-114910 | A | 10 August 2021 | US 2021/0222106 A1 claims, paragraphs [0072]-[0187], [0208], fig. 1-7 EP 3854587 A1 | | | |
| WO | 2019/168118 | A1 | 06 September 2019 | US 2020/0369998 A1 claims, examples, fig. 1-11B EP 3744678 A1 CN 111788147 A | | | |
| JP | 2020-188723 | A | 26 November 2020 | (Family: none) | | | |
| JP | 2018-102236 | A | 05 July 2018 | US 2018/0179481 A1 whole document EP 3342852 A2 | | | |
| WO | 2015/159821 | A1 | 22 October 2015 | US 2017/0137767 A1 paragraph [0055] EP 3133145 A1 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021138137 A

- JP 2018102236 A